# EUROPEAN PATENT APPLICATION

(11) **EP 1 421 945 A1**
(43) Date of publication of application: **26.05.2004**
(21) Application number: 02755899.8
(22) Date of filing: 09.08.2002
(51) Int. Cl.: A61K 35/74, A61K 31/702, A61P 3/06, A61P 35/00, A61P 43/00

(54) **BILE ACID ABSORBENT/ADSORBENT**

(30) Priority: 10.08.2001 JP 2001244742
(71) Applicant: Hokkaido Technology Licensing Office Co., Ltd., Sapporo-Shi, Hokkaido 060-0807 (JP); NIPPON BEET SUGAR MFG. CO., LTD., Tokyo 104-0031 (JP); The Hokuren Federation Of Agricultural Cooperatives, Sapporo, Hokkaido 060-8651 (JP)
(72) Inventor: YOKOTA, Atsushi, Sapporo-shi, Hokkaido 004-0881 (JP); TOMITA, Fusao, Sapporo-shi, Hokkaido 063-0824 (JP); SAYAMA, Kouji, Obihiro-shi, Hokkaido 080-0836 (JP); HUKUMORI, Yasunori, Chuo-ku, Sapporo-shi,Hokkaido 060-0012 (JP)
(74) Representative: HOFFMANN EITLE
(86) International application number: PCT/JP2002/008167
(87) International publication number: WO 2003/013559

(57) **Abstract**

The present invention provides a bile acid absorbent/adsorbent comprising as active ingredients a lactic acid bacterium having a property of incorporating bile acids into the cell in the presence of an oligosaccharide and of no releasing them out of the cells, and the oligosaccharide. The lactic acid bacterium includes *Lactobacillus salivarius* ssp. *salicinius* strain JCM1044, *Bifidobacterium breve* strain JCM1192, etc. The oligosaccharide includes raffinose, kestose, nystose, trehalose, etc. The bile acid adsorbent is in a form of oral formulation, or eatables and drinkables, etc. The bile acid absorbent/adsorbent can be utilized as cholesterol lowering agents or colorectal cancer inhibitors.

## Description

### Technical Field

The present invention relates to a bile acid absorbent/adsorbent, and more particularly to a bile acid absorbent/adsorbent comprising as active ingredients a lactic acid bacterium adsorbing or absorbing bile acids and an oligosaccharide. According to the present invention, bile acids can be adsorbed on bifidobacterial and lactic acid bacterial cells and eliminated from the body. Therefore, the present invention regulates partially a so-called enterohepatic circulation of bile acids, and thus permits a lowering of serum cholesterol concentration in the body. Further, the present invention permits a lowering of colorectal cancer risk due to a lowering of secondary bile acids concentration in the large intestine. Therefore, the present invention makes it possible to provide cholesterol-lowering agents or colorectal cancer inhibitors.

### Background Art

Bile acids in human are composed of 40% of cholic acid, 30% of chenodeoxycholic acid and 20% of deoxycholic acid, and play an important role in digestion and absorption of fatty acids.

On the other hand, the enterohepatic circulation acts as a cycle for efficiently utilizing bile acids. It is a cycle for reabsorbing used bile acids in the small intestine and large intestine and reutilizing them. It is reported that the rate of reutilization is 95% or more in healthy individuals, and 90% or 10% of the reutilized bile acids is present in the small intestine or large intestine, respectively (M. Kanemura (1982): Surgery Today, 83: 677-690). Bile acids are produced by biosynthesis in the liver from cholesterol in blood by quantity required depending on the amount of bile acids that are not reabsorbed in the large intestine. The above is an outline of the enterohepatic circulation of bile acids.

Therefore, if the enterohepatic circulation is inhibited, for example if bile acids can be adsorbed on something for inhibiting the reabsorption of bile acids from the intestinal tract, the amount of eliminated bile acids is increased thereby facilitating the synthesis of required bile acids from serum cholesterol to result in a lowering of serum cholesterol concentration. Form this idea, a concept called as bile acid adsorbents is proposed, and they are used in medical services under the name of cholestyramine (Yutaka MIZUSHIMA, Akimasa MIYAMOTO: Present-Day Therapeutics (1995) 394). However, the drug is essentially an ion exchange resin, requires a strict prescription and thus is not suitable for applications under health insurance.

Considering the present state of the art as mentioned above, the present inventors focus on the facts that elimination or removal of bile acids from the body results in a lowering of cholesterol concentration and an improvement in human serum lipid metabolism, and that a lowering of secondary bile acid concentration in the large intestine results in a lowering of colorectal cancer risk, and they fully realize the importance of the technique for reducing bile acids or eliminating them.
Consequently, an object of the present invention is to develop a new system for reducing bile acids or eliminating them. The system needs to be a completely new type of bile acid eliminating system giving strong consideration not only to the efficiency but also to the safety.

### Disclosure of Invention

The present inventors focus on microorganism from the standpoint of the aspect of safety after examining from several aspects, and found for the first time that there are present lactic acid bacteria which take up bile acids and do not eliminate them, or lactic acid bacteria which absorb or adsorb bile acids and do not release them, and that the function is operated and/or promoted in the presence of an oligosaccharide. The further study based on the new and valuable knowledge has led finally to the completion of the present invention.

Therefore, the present invention relates to the following aspects:
- as a first aspect, a bile acid absorbent/adsorbent characterized by comprising as active ingredients a lactic acid bacterium absorbing bile acids and an oligosaccharide;
- as a second aspect, a bile acid absorbent/adsorbent characterized by comprising as active ingredients a lactic acid bacterium absorbing and adsorbing bile acids in the presence of an oligosaccharide, and the oligosaccharide;
- as a third aspect, the bile acid absorbent/adsorbent as set forth in the first or second aspect characterized in that the lactic acid bacterium is at least one selected from *Lactobacillus* and/or *Bifidobacterium;*
- as a fourth aspect, the bile acid absorbent/adsorbent as set forth in any one of the first to third aspects characterized in that the oligosaccharide is a low digestible oligosaccharide;
- as a fifth aspect, the bile acid absorbent/adsorbent as set forth in any one of the first to third aspects characterized in that the oligosaccharide is at least one selected from raffinose, kestose, nystose and trehalose;
- as a sixth aspect, a cholesterol-lowering agent characterized by comprising as active ingredients a lactic acid bacterium absorbing bile acids and an oligosaccharide;
- as a seventh aspect, a cholesterol-lowering agent characterized by comprising as active ingredients a lactic acid bacterium absorbing and adsorbing bile acids in the presence of an oligosaccharide, and the oligosaccharide;
- as an eighth aspect, the cholesterol-lowering agent as set forth in the sixth or seventh aspect characterized in that the lactic acid bacterium is at least one selected from *Lactobacillus* and/or *Bifidobacterium*;
- as a ninth aspect, the cholesterol-lowering agent as set forth in any one of the sixth to eighth aspects characterized in that the oligosaccharide is a low digestible oligosaccharide;
- as a tenth aspect, the cholesterol-lowering agent as set forth in any one of the sixth to eighth aspects characterized in that the oligosaccharide is at least one selected from raffinose, kestose, nystose and trehalose;
- as an eleventh aspect, a colorectal cancer inhibitor characterized by comprising as active ingredients a lactic acid bacterium absorbing bile acids and an oligosaccharide;
- as a twelfth aspect, a colorectal cancer inhibitor characterized by comprising as active ingredients a lactic acid bacterium absorbing and adsorbing bile acids in the presence of an oligosaccharide, and the oligosaccharide;
- as a thirteenth aspect, the colorectal cancer inhibitor as set forth in the eleventh or twelfth aspect characterized in that the lactic acid bacterium is at least one selected from *Lactobacillus* and/or *Bifidobacterium*;
- as a fourteenth aspect, the colorectal cancer inhibitor as set forth in any one of the eleventh to thirteenth aspects characterized in that the oligosaccharide is a low digestible oligosaccharide; and
- as a fifteenth aspect, the colorectal cancer inhibitor as set forth in any one of the eleventh to thirteenth aspects characterized in that the oligosaccharide is at least one selected from raffinose, kestose, nystose and trehalose.
   The present invention relates to a bile acid absorbent/adsorbent, a cholesterol lowering-lowering agent and a colorectal cancer inhibitor comprising lactic acid bacteria absorbing and/or adsorbing bile acids (the bacteria include any lactic acid bacteria that can retain bile acids therein, such as lactic acid bacteria absorbing and/or adsorbing bile acids, lactic acid bacteria adhering bile acids thereon, lactic acid bacteria taking up bile acids thereinto, and the like, and further suitably lactic acid bacteria that tend not to eliminate the bile acids taken up out of the cells) as an active ingredient. The present invention results in the function of lactic acid bacteria and/or the promotion of the function as mentioned above by making the lactic acid bacteria and an oligosaccharide present together in the body.

### Brief Description of Drawings

Fig. 1 shows activities of cholic acid uptake by *Lactobacillus salivarius* ssp. *salicinius* strain JCM1044 in the presence of raffinose (Raf);
Fig. 2 shows activities of cholic acid uptake by *Bifidobacterium breve* strain JCM1192 in the presence of raffinose;
Fig. 3 shows activities of chenodeoxycholic acid uptake by the above-mentioned strain JCM1192 in the presence of raffinose;
Fig. 4 shows activities of cholic acid uptake by the above-mentioned strain JCM1044 in the presence of 1-kestose (Kes);
Fig. 5 shows activities of cholic acid uptake by the above-mentioned strain JCM1192 in the presence of 1-kestose; and
Fig. 6 shows activities of chenodeoxycholic acid uptake by the above-mentioned strain JCM1192 in the presence of 1-kestose.

### Best Mode for Carrying Out the Invention

In the present invention, a lactic acid bacterium or two or more lactic acid bacteria having the above-mentioned property are appropriately used in order to absorb and adsorb bile acids in the body thereby reducing the amount of bile acids. A preferable example includes lactic acid bacteria having a property of taking up bile acids into the cells in the presence of an oligosaccharide and no releasing them out of the cells.

The lactic acid bacteria that can be used in the present invention are any lactic acid bacteria as mentioned above, and bacteria belonging to *Lactobacillus* and/or *Bifidobacterium* can be appropriately used. These lactic acid bacteria include for example strains registered and conserved in Japan Collection of Microorganisms (JCM) of RIKEN (The Institute of Physical and Chemical Research).

Among strains registered and conserved in Japan Collection of Microorganisms (JCM) of RIKEN, bacteria belonging to *Lactobacillus* include *Lactobacillus delbruekii subsp. bulgaricus* strain JCM1002, *Lactobacillus acidophilus* strain JCM1028, *Lactobacillus acidophilus* strain JCM1034, *Lactobacillus salivarius* ssp. *salicinus* strain JCM1044, *Lactobacillus helveticus* strain JCM1062, *Lactobacillus buchneri* strain JCM1115, *Lactobacillus mali* strain JCM1116, *Lactobacillus plantarum* strain JCM1149, *Lactobacillus sakei* strain JCM1157 and the like, and bacteria belonging to *Bifidobacterium* include *Bifidobacterium breve* strain JCM1192, *Bifidobacterium bifidum* strain JCM1255, *Bifidobacterium infantis* strain JCM1222 and the like.

In the meantime, any of these lactic acid bacteria are recorded in the JCM catalogue of strains, and freely available and obtainable.

Particularly preferable lactic acid bacteria are the above-mentioned *Lactobacillus salivarius* ssp. *salicinus* strain JCM1044 and *Bifidobacterium breve* strain JCM1192 that are deposited on August 7, 2002 with independent administrative agency the International Patent Organism Depositary of Advanced Industrial Science and Technology under International deposit numbers PERM BP-8145 and FERM BP-8144, respectively.

Although these lactic acid bacteria have a property of absorbing and adsorbing bile acids, the property is not revealed until the bacteria are in the presence of an oligosaccharide and/or it is further promoted in the presence of an oligosaccharide. Therefore, the bile acid absorbent/adsorbent according to the present invention may be orally administered in a form of a formulation comprising lactic acid bacteria and an oligosaccharide, or the bile acid absorbent/adsorbent may be given by orally administering lactic acid bacteria and an oligosaccharide prepared separately at the same time, by orally administering only lactic acid bacteria in a case where an oligosaccharide is present in the body, particularly in the intestine, or by orally administering only an oligosaccharide to the contrary. In a short, the present invention includes all forms for administration and is not limited to only a case where lactic acid bacteria are present along with an oligosaccharide when it is administered. In the meanwhile, although the present invention will be explained on the bile acid absorbent/adsorbent as a representative example hereinafter, the explanation is applied similarly to the cholesterol-lowering agent or the colorectal cancer inhibitor.

The bile acid absorbent/adsorbent according to the present invention can be appropriately prepared by using common methods for formulation. It can be prepared by adding an excipient, a binder, a disintegrator, a lubricant, a corrigent, a solubilizer, an emulsifier, a coating agent or other additives of common use to the active ingredients, and then formulating into a powder, a granule, a capsule, a tablet, a liquid or the like. In the meantime, it can be formulated into an enteric coating drug according to a conventional method.

In addition, the active ingredients can be formulated with eatable or drinkable ingredients into a solid form (a powder, a granule, etc.), a paste form, a liquid form or an emulsion form, a milk product, such as fermented milk, cheese or butter; a drink, such as drinkable yogurt or lactic acid bacteria beverage; confectionery and bakery products, such as a butter cake, or other forms of eatable or drinkable goods.

As the active ingredients, lactic acid bacteria and an oligosaccharide are used (it is not always required to formulate both ingredients simultaneously). The lactic acid bacteria may be isolated lactic acid bacteria themselves, or a lactic acid bacteria-containing product or the processed product thereof that is included in the present invention.

The lactic acid bacteria containing product includes a suspension of lactic acid bacteria, a cultured product of lactic acid bacteria (including a bacterial body (cell), a cultured supernatant or medium components), a cultured fluid of lactic acid bacteria prepared by removing solid components from a cultured product of lactic acid bacteria, lactic acid bacteria beverage, fermented milk containing lactic acid bacteria-fermented eatables and drinkables, such as sour milk or yogurt.

The processed product includes the concentrated product, pasted product, dried product (spray-dried product, freeze-dried product, vacuum-dried product, drum-dried product, etc.), dilution and the like of the lactic acid bacteria, lactic acid bacteria containing product or fermented milk.

The above is similarly applied to the oligosaccharide being the other active ingredient, a purified product or a dried product of the oligosaccharide can be used, and further in a case where oligosaccharide prepared by fermentation is used, an oligosaccharide containing product or the processed product thereof can be used in a similar manner as above.

As the oligosaccharide, raffinose, kestose, nystose, trehalose, lactulose and several other oligosaccharides can be used, and commercially available products can be also used.

Raffinose used in the present invention includes, for example one prepared from roots of sugar beet by known methods (e.g., Japanese Patent Laid-open No. Sho 54-49345) or a crude product prepared and commercially available as soybean oligosaccharides (Japan Food Science, Vol. 26, No. 10, pp. 56-64, 1987), and they can be directly used as "an oligosaccharide containing raffinose". Further, it may be raffinose prepared directly from soybean whey by known methods (e.g., Japanese Patent Laid-open No. Sho 59-179064).

In addition, lactulose used in the present invention may be prepared by alkaline isomerization of lactose according to known methods, and may be used in any dosage form of syrup, powder, granule and the like. Because of a small amount of by-product, for example lactulose prepared by the method disclosed in Japanese Patent Publication No. Sho 52-21063 is preferable.

Further, fructooligosaccharide used in the present invention includes for example 1-kestose, nystose and the like, and can be prepared from a sucrose solution by known methods (e.g., Japanese Patent Laid-open No. Hei 8-173109, Japanese Patent Publication No. Sho 59-53834, etc.).

In addition, examples of galactooligosaccharides are compounds of the following formula:

Gal-(Gal)ₙ-Glc

wherein Gal is a galactose residue, Glc is a glucose residue and n is an integer of 1 to 4, and they can be prepared from a lactose solution by know methods (e.g., Japanese Patent Publication No. Sho 58-20266, etc.).

Examples of commercially available oligosaccharides include trehalose (Trehanoinochi: trade name of H+B Lifescience Co., Ltd.), raffinose (produced by Nippon Beet Sugar Manufacturing Co., Ltd.), lactulose (produced by Morinaga Milk Industry Co., Ltd.), galactoofigosaccharide (produced by Nisshin Sugar Manufacturing Co., Ltd.), lactosucrose (produced by Hayashibara Shoji, Inc.), fructooligosaccharide (produced by Meiji Seika Kaisha, Ltd.), isomaltooligosaccharide (produced by Hayashibara Shoji, Inc.), xylooligosaccharide (produced by Suntory Limited) and the like.

Further, oligosaccharides prepared by the above-mentioned methods may be used. As to oligosaccharides comprising a mixture of 1-kestose, nystose and F-nystose, a manufacturing process by utilizing an enzyme derived from *Aspergillus niger* (Japanese Patent Laid-open No. Sho 61-268190) and a manufacturing process by utilizing an enzyme derived from *Aureobasidium pullulans* (Japanse Patent Laid-open No. Sho 57-166981) are proposed and performed, and the above-mentioned mixture can be used in the present invention. Meanwhile, in particular, 1-kestose among these oligosaccharides can be utilized by a number of lactic acid bacteria (H. Hidaka et al. "Effect of fructooligosaccharides on intestinal flora, intestinal flora and food factor" (ed. T. Mitsuoka), pp. 39-66, Japan Scientific Societies Press, Tokyo, 1984), and therefore it is preferable to use 1-kestose crystals prepared by a combination of a preparation of 1-kestose by fermentation of *Scopulariopsis brevicaulis* (Japanese Patent Publication No. Hei 4-41600) or a process for enrichment of 1-kestose by using an enzyme derived from *Eurotium repens* and a chromatographic separation (Japanese Patent Laid-open No. 2000-232878) and a method for crystallizing 1-kestose (Japanese Patent Publication No. Hei 6-70075). In addition, according to the object, it is also able to prepare and use nystose crystals by using a method for crystallizing nystose (Japanese Patent No. 2640577).

The above-mentioned active ingredients (lactic acid bacteria, oligosaccharides) generally exert their effect when they reach the intestine. However, if desired they may be formulated into an enteric coating drug in order to make sure that they are delivered to the intestine. In addition, it is able to utilize lactic acid bacteria already existing in the intestine themselves. In this case, lactic acid bacteria need not be administered, and it is sufficient to administer only an oligosaccharide.

As oligosaccharide, any of low digestible oligosaccharides or high digestible oligosaccharides may be used. Meanwhile, as the oligosaccharides need reach the intestine, in case where oligosaccharides, such as high digestible oligosaccharides which cannot reach the intestine are used or only a low amount of those reach the intestine, a countermeasure, such as a formulation into an enteric coating drug should be laid down.

Further, low digestible oligosaccharides, such as raffinose, 1-kestose, nystose, etc. generally reach the intestine without decomposition in contrast with monosaccharides or high digestible oligosaccharides. For example, in case where raffinose is used, even if it is decomposed, this is not necessarily disadvantages as the decomposition thereof leads to melibiose being a bifidus factor. Consequently, the oligosaccharides exert not only the intended effect but also the inherent effects as a bifidus factor or a factor in immunological enhancement. In addition, the oligosaccharides may be used in a lower amount than monosaccharides, and the present invention is excellent also in this point.

The above-mentioned measures according to the present invention induces an active synthesis of bile acids from serum cholesterol, thereby lowering cholesterol concentration. Further, an increase of bile acids in the large intestine acts promotively in the development of colorectal cancer (K. Kanazawa, Journal of Japan Clinical Medicine, Vol. 42, 1696-1700), and therefore the action of absorbing bile acids according to the method in present invention permits a lowering of colorectal cancer risk. Thus, the present invention provides cholesterol-lowering agents and colorectal cancer inhibitors.

Hereinafter, examples and preparation examples are indicated and the present invention is explained in more detail. Meanwhile, the activity of bile acid uptake was measured according to the method described below.

Lactic acid bacteria were cultured in a commercially available MRS medium overnight. The composition of the medium was as follows:
MRS medium (1 liter)
- Peptone 10 g
- Meat extract 10 g
- Yeast extract 5 g
- Dipotassium hydrogenphosphate 2 g
- Diammonium citrate 2 g
- Glucose 20 g
- Tween 80 1 g
- Sodium acetate 5 g
- Magnesium sulfate (hepta hydrate) 0.58 g
- Manganese sulfate (tetra hydrate) 0.28 g.

Next, a second generation culture was prepared, and cells were harvested by centrifugation when the culture fluid exhibited the turbidity (absorbance at a wavelength of 660 nm) of 0.6. The harvested cells were washed with a buffer (pH 7.0) of 50 mM potassium phosphate containing 1 mM magnesium sulfate, and then the washed cells were re-suspended in a buffer comprising the same composition. The resulting cell suspension was allowed to stand for 30 minutes to make the cell membrane of the cells the state of deactivation. Then, the cells were harvested by centrifuging the suspension and washed with a buffer (pH 7.0) of 50 mM potassium phosphate containing 1 mM magnesium sulfate. Thereafter, the washed cells were re-suspended in a buffer comprising the same composition, and the cell suspension was adjusted so as to have the turbidity of 10.

To 96 µl of the cell suspension prepared as above, 2 µl of 6 mM cholic acid solution (cholic acid (chenodeoxycholic acid) labeled with ¹⁴C, 16 mCi/mmol) was added, and after allowing the resulting suspension to stand for 15 minutes; 0.1 µl of 0.33 M oligosaccharide solution was added thereto, and the measurement of the activity of bile acid uptake was started. After a predetermined time, the reaction was stopped by adding 3 ml of a buffer of 100 mM potassium phosphate containing 100 mM lithium hydrochloride, and then the reaction solution was filtered through a cellulose acetate membrane (pore size: 0.45 µm), and the radioactivity left on the membrane was measured by a scintillation counter. In addition, as a control, the radioactivity of a sample adding no oligosaccharide was measured similarly.

### Example 1

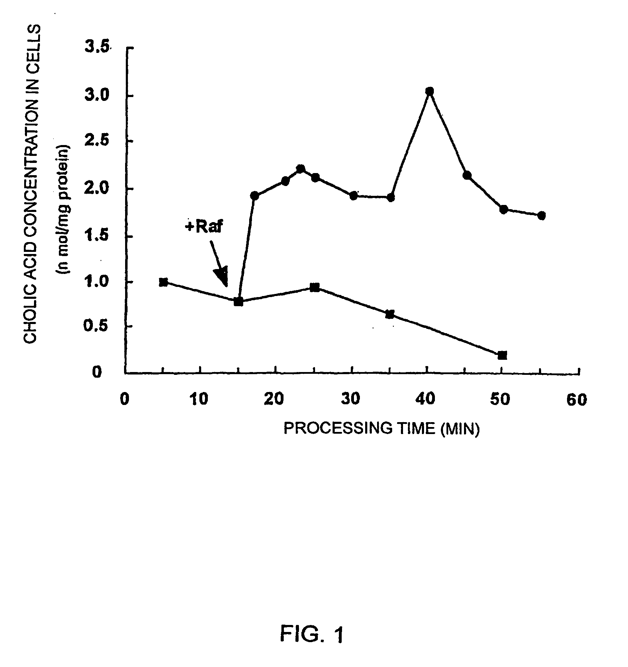

As to *Lactobacillus salivarius* ssp. *salicinius* strain JCM1044, the activity of bile acid uptake was measured. The result is shown in Fig. 1. In the figure, sections (test sections) where oligosaccharides were used are shown in black dots, and sections where they were not used are shown in black squares (hereinafter referred in a similar manner).

Raffinose was added at the point of 15 minutes after the reaction was started. In the cell suspension in which raffinose was not added, bile acid was equilibrated into the cells, and thereby cholic acid concentration in the cells was unchanged. On the other hand, in the cell suspension in which raffinose was added, bile acids were rapidly incorporated into the cells, and thereby cholic acid concentration in the cells rose to about 8 times that of the outside of the cells.

After the point (the point of 40 minutes after the reaction was started) when it was considered that raffinose added into the cell suspension was almost consumed, cholic acid was passively eliminated, thereby gradually lowering the cholic acid concentration in the cells.

### Example 2

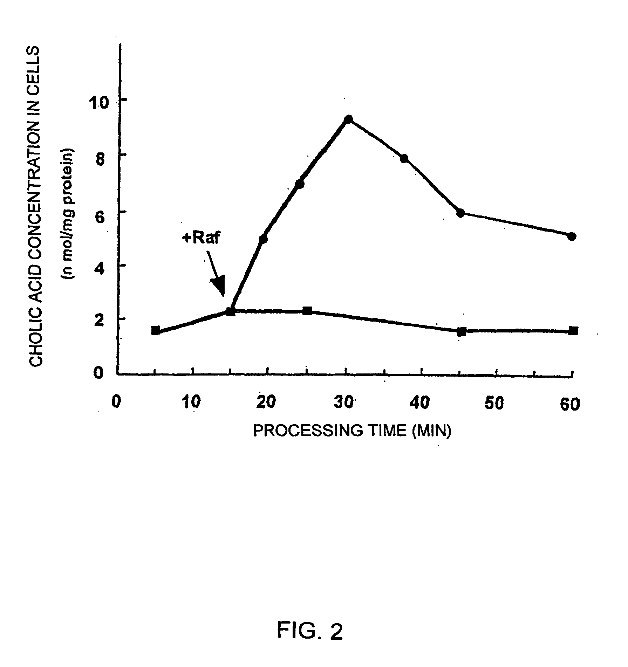

As to *Bifidobacterium breve* strain JCM1192, the activity of bile acid uptake was measured. The result is shown in Fig. 2.

Raffinose was added at the point of 15 minutes after the reaction was started. In the cell suspension in which raffinose was not added, bile acid was equilibrated into the cells, and thereby cholic acid concentration in the cells was unchanged, similarly to Example 1. On the other hand, in the cell suspension in which raffinose was added, bile acids were rapidly incorporated into the cells, and thereby cholic acid concentration in the cells rose to about 30 times that of the outside of the cells.

After the point (the point of 60 minutes after the reaction was started) when it was considered that raffinose added into the cell suspension was almost consumed, cholic acid was passively eliminated, thereby gradually lowering the cholic acid concentration in the cells.

### Example 3

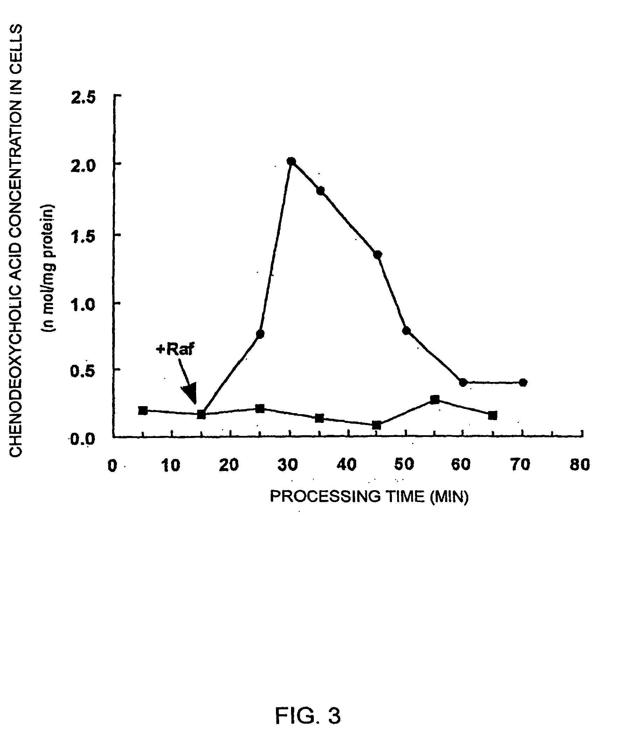

As to *Bifidobacterium breve* strain JCM1192, the activity of chenodeoxycholic acid uptake was measured. The result is shown in Fig. 3.

Raffinose was added at the point of 15 minutes after the reaction was started. In the cell suspension in which raffinose was not added, chenodeoxycholic acid was equilibrated into the cells, and thereby chenodeoxycholic acid concentration in the cells was unchanged. On the other hand, in the cell suspension in which raffinose was added, chenodeoxycholic acids were rapidly incorporated into the cells, and thereby chenodeoxycholic acid concentration in the cells rose to about 6 times that of the outside of the cells.

After the point (the point of 30 minutes after the reaction was started) when it was considered that raffinose added into the cell suspension was almost consumed, chenodeoxycholic acid was passively eliminated, thereby gradually lowering the chenodeoxycholic acid concentration in the cells.

### Preparation Example 1

### Preparation of kestose crystal

### (1) Production of an enzyme derived from Eurotium repens

In a jarfermentor, was prepared 200 L of a medium being a solution of pH 6 comprising 30% of sucrose as carbon source, 1.5% of yeast extract as nitrogen source, 0.2% of calcium phosphate and 0.2% of magnesium sulfate, and sterilized, and after that, the medium was inoculated with *Eurotium repens* harvested from shaken culture in a medium having the same composition for 48 hours in advance, the jarfermentor was bubbled with air at a rate of 1/2 VVM, and cells were separated through a filter to obtain 6 kg of wet cells. The wet cells were subjected to the following reaction as crude enzymes.

### (2) Enzyme reaction

A reaction solution having a sucrose concentration of 70% (W/V) was prepared in an amount of 1 m³, and adjusted to pH 6. Then, 5 kg of the above-mentioned wet cells was added to the solution and reacted at a reaction temperature of 55°C with stirring for 16 hours. The resulting reacted solution had a sugar composition of 30% of 1-kestose, 9% of nystose, 20% of glucose, 2% of fructose and 33% of sucrose based on the all sugars. The above-mentioned reaction was repeated 5 times, and then 1-kestose was isolated and purified through the chromatographic separation described below.

### (3) Chromatographic separation

The reacted solution mentioned above was subjected to a chromatographic separation by using a simulated moving bed chromatographic separation device (as desorbent, a cation exchange resin UBK 530 provided by Mitsubishi Chemical Corporation was used) by which the solution was separated into an oligosaccharide fraction mainly comprising 1-kestose and other fraction mainly comprising sucrose and glucose. The separation condition was as follows: operation load of 0.05 v/v.h and amount of used water of 5.33 (D/F). The resulting oligosaccharide fraction had a sugar composition of 72% of 1-kestose, 19% of nystose and 2% of sucrose, and a recovery of 1-kestose was 94%.

Next, the oligosaccharide fraction was concentrated to 65% (W/W), and the resulting concentrated liquid was subjected to a separation into 1-kestose fraction and nystose fraction mainly comprising nystose by altering separation condition of the chromatographic separation device. The separation condition as follows: operation load of 0.04 v/v.h and amount of used water of 5.00 (D/F). The resulting kestose fraction had a sugar composition of 89% of 1-kestose, 3% of nystose and 3% of sucrose, and a recovery of 1-kestose was 63%. Consequently, 900 L (70% (W/W) of an operated liquid was obtained. Crystallization process was carried by using the liquid as mother liquor and thereby rising the purity.

### (4) Crystallization process

A part of the kestose fraction was introduced into a 500 L crystallization can, and seeded with crystals previously prepared at a point when the fraction was concentrated to 89% (W/W), and then sugar crystallization was carried out 85°C for 3 hours. After that, an auxiliary crystallization was carried out by controlling a temperature from 80°C to 65°C for 17 hours, and then crystals was collected by a centrifuge, and dried and cooled. The resulting crystallized product had a weight of 453 kg, a recovery of 50% based on kestose in the mother liquor and a purity of 98%.

### Example 4

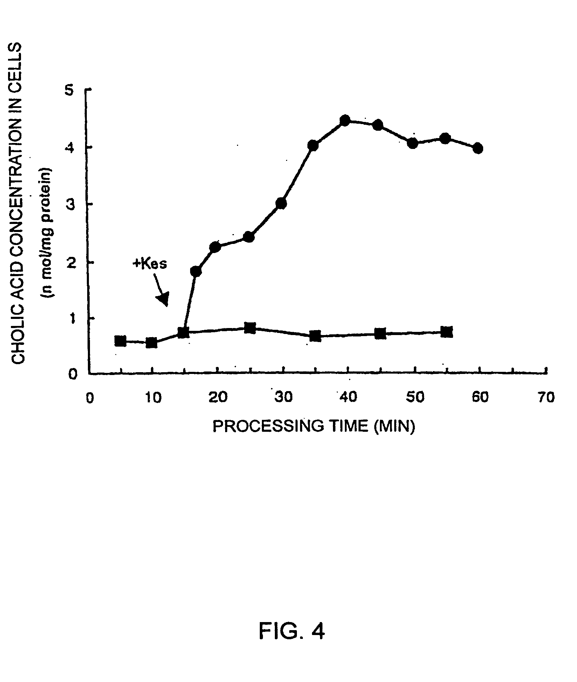

As to *Lactobacillus salivarius* ssp. *salicinius* strain JCM1044, the activity of bile acid uptake was measured. The result is shown in Fig. 4. Meanwhile, the crystallized product prepared in Preparation Example 1 was used as 1-kestose.

1-Kestose was added at the point of 15 minutes after the reaction was started. In the cell suspension in which 1-kestose was not added, bile acid was equilibrated into the cells, while in the cell suspension in which 1-kestose was added; bile acids were rapidly incorporated into the cells, and thereby cholic acid concentration in the cells rose to about 13 times that of the outside of the cells. After the point (the point of 40 minutes after the reaction was started) when it was considered that 1-kestose added into the cell suspension was almost consumed, there occurred a tendency for passive elimination of cholic acid.

### Example 5

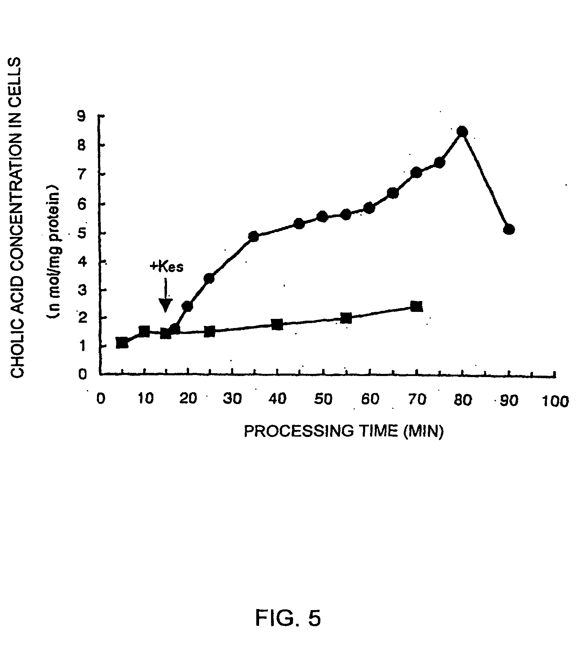

As to *Bifidobacterium breve* strain JCM1192, the activity of cholic acid uptake was measured. The result is shown in Fig. 5. 1-Kestose was added at the point of 15 minutes after the reaction was started. In the cell suspension in which 1-kestose was not added, bile acids were not incorporated into the cells, while in the cell suspension in which 1-kestose was added, bile acids were rapidly incorporated into the cells, and thereby cholic acid concentration in the cells rose to about 24 times that of the outside of the cells. After the point (the point of 80 minutes after the reaction was started) when it was considered that 1-kestose added into the cell suspension was almost consumed, cholic acid was passively eliminated. Further, also as to *Bifidobacterium infantis* strain JCM1222, it was confirmed that cholic acid was incorporated into the cells in a nystose added-cell suspension.

### Example 6

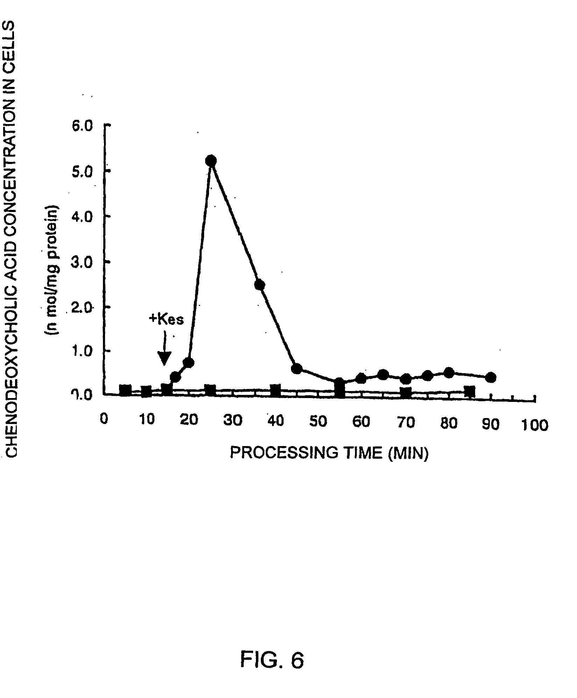

As to *Bifidobacterium breve* strain JCM1192, the activity of chenodeoxycholic acid uptake was measured. The result is shown in Fig. 6.

1-Kestose was added at the point of 15 minutes after the reaction was started. In the cell suspension in which 1-kestose was not added, chenodeoxycholic acid was not incorporated into the cells, while in the cell suspension in which 1-kestose was added, chenodeoxycholic acid was rapidly incorporated into the cells, and thereby chenodeoxycholic acid concentration in the cells rose to about 15 times at the most that of the outside of the cells. After the point (the point of 30 minutes after the reaction was started) when it was considered that 1-kestose added into the cell suspension was almost consumed, chenodeoxycholic acid was passively eliminated, thereby gradually lowering the chenodeoxycholic acid concentration in the cells. However, the concentration came to equilibrium at a point of about 2 times.

### Example 7

### Preparation of kestose tablet

A table having a weight of 150 mg was prepared by mixing 1-kestose, dextrin and vegetable fat in a proportion of 50%, 30% and 20%, respectively and using a tableting machine. The resulting tablet had a strength of 4.2 kg.

### Example 8

### Preparation of tablet comprising Bifidobacterium breve strain JCM1192 and the above-mentioned oligosaccharide

MRS medium was inoculated with *Bifidobacterium breve* strain JCM1192, and it was incubated statically at 37°C for 24 hours. After the incubation was completed, cells was harvested by centrifugation. The cells were mixed into a dispersion medium comprising 5% of sucrose, 5% of soluble starch, 5% sodium glutamate and 1% of magnesium sulfate hepta hydrate, and the resulting mixture was adjusted to pH 7.0, then the mixture was lyophilized.

100 mg of 1-kestose or nystose, 60 mg of dextrin, 18 mg of polyvinyl pyrrolidone K25 and 0.8 mg of magnesium stearate were added to 2 mg of dried cell powder prepared as mentioned above, and the mixture was uniformly mixed, and compressed by a tableting machine into two kinds of tablets having a weight of 200 mg per tablet and comprising oligosaccharides and bifid bacteria.

### Industrial Applicability

According to the present invention, it was found that bile acids can be incorporated into bifid bacteria or lactic acid bacteria in the presence of an oligosaccharide and are not excluded again. Thus, as oligosaccharides can be used as bile acid uptake inducer, serum cholesterol concentration or colorectal cancer risk can be lowered by using these sugars together with the above-mentioned bifid bacteria or lactic acid bacteria or by using only these sugars in expectation of increase of indigenous bacteria in the intestine. In particular, the development of cancer is depressed even if a low amount of the active ingredients is used. Therefore, the active ingredients of the present invention can be administered in a form of medicine or eatables and drinkables for prevention of the development of cancer or prevention of relapse after surgery.

## Claims

1. A bile acid absorbent/adsorbent **characterized by** comprising as active ingredients a lactic acid bacterium absorbing bile acids and an oligosaccharide.

2. A bile acid absorbent/adsorbent **characterized by** comprising as active ingredients a lactic acid bacterium absorbing and adsorbing bile acids in the presence of an oligosaccharide, and the oligosaccharide.

3. The bile acid absorbent/adsorbent according to claim 1 or 2, **characterized in that** the lactic acid bacterium is at least one selected from *Lactobacillus* and/or *Bifidobacterium*.

4. The bile acid absorbent/adsorbent according to any one of claims 1 to 3, **characterized in that** the oligosaccharide is a low digestible oligosaccharide.

5. The bile acid absorbent/adsorbent according to any one of claims 1 to 3, **characterized in that** the oligosaccharide is at least one selected from raffinose, kestose, nystose and trehalose.

6. A cholesterol-lowering agent **characterized by** comprising as active ingredients a lactic acid bacterium absorbing bile acids and an oligosaccharide.

7. A cholesterol-lowering agent **characterized by** comprising as active ingredients a lactic acid bacterium absorbing and adsorbing bile acids in the presence of an oligosaccharide, and the oligosaccharide.

8. The cholesterol-lowering agent according to claim 6 or 7, **characterized in that** the lactic acid bacterium is at least one selected from *Lactobacillus* and/or *Bifidobacterium*.

9. The cholesterol-lowering agent according to any one of claims 6 to 8, **characterized in that** the oligosaccharide is a low digestible oligosaccharide.

10. The cholesterol-lowering agent according to any one of claims 6 to 8, **characterized in that** the oligosaccharide is at least one selected from raffinose, kestose, nystose and trehalose.

11. A colorectal cancer inhibitor **characterized by** comprising as active ingredients a lactic acid bacterium absorbing bile acids and an oligosaccharide.

12. A colorectal cancer inhibitor **characterized by** comprising as active ingredients a lactic acid bacterium absorbing and adsorbing bile acids in the presence of an oligosaccharide, and the oligosaccharide.

13. The colorectal cancer inhibitor according to claim 11 or 12, **characterized in that** the lactic acid bacterium is at least one selected from *Lactobacillus* and/or *Bifidobacterium*.

14. The colorectal cancer inhibitor according to any one of claims 11 to 13, **characterized in that** the oligosaccharide is a low digestible oligosaccharide.

15. The colorectal cancer inhibitor according to any one of claims 11 to 13, **characterized in that** the oligosaccharide is at least one selected from raffinose, kestose, nystose and trehalose.
